# FASCICULE DE BREVET EUROPEEN

(11) **EP 2 724 675 B1**
(45) Date de publication et mention de la délivrance du brevet: **06.07.2016**
(21) Numéro de dépôt: 13189562.5
(22) Date de dépôt: 21.10.2013
(51) Int. Cl.: A61B 17/15, A61B 17/17

(54) **Gabarit de pose d'un implant d'articulation d'une prothèse d'épaule sur une tête humérale**
Einsatzschablone für ein Gelenkimplantat einer Schulterprothese auf einem Humeruskopf
Template for installing an articulation implant for a shoulder prosthesis on a humeral head

(30) Priorité: 26.10.2012 FR 1260203
(43) Date de publication de la demande: 30.04.2014
(73) Titulaire: Aston Medical, 42000 Saint Etienne (FR)
(72) Inventeur: Gonzalvez, Martin, 21000 Dijon (FR); Trouilloud, Pierre, 21000 Dijon (FR); Colombier, Michel, 34000 Montpellier (FR)
(74) Mandataire: Cabinet Laurent & Charras

(56) Documents cités:
- WO-A2-2012/021241
- US-A1- 2009 264 889
- US-A1- 2012 041 446
- US-A1- 2012 143 267

## Description

L'invention se rattache au secteur technique des instruments à usage unique personnalisés au patient pour la pause d'implants orthopédiques notamment pour une prothèse de l'articulation de l'épaule. L'état de la technique le plus proche est donné par le document WO 2012/021241 A2, qui définit le préambule de la revendication 1.

Plus particulièrement, l'invention concerne un gabarit huméral sur mesure pour la pause d'une prothèse d'épaule notamment du type inversée.

On rappelle que par prothèse d'articulation d'épaule inversée, on entend une prothèse dont la tête hémisphérique d'articulation, en tant que telle, est fixée au niveau de la cavité glénoïdienne pour coopérer avec une cupule ou platine de forme complémentaire, fixée sur une tige humérale. Autrement dit, dans ce type de prothèse, l'implant glénoïdien comprend une tête d'articulation hémisphérique tandis que l'implant huméral comprend une cupule ou platine.

Il est désormais admis que la fixation et le positionnement des implants prothétiques s'avèrent particulièrement importants pour la réussite du fonctionnement biomécanique de la prothèse d'épaule et de sa durée de vie.

Une solution avantageuse pour la pose d'une prothèse d'épaule ressort de l'enseignement du document FR 2 967 047 dont le demandeur de la présente est également titulaire.

Ce document concerne un gabarit de pose d'une prothèse d'épaule sur une glène en planifiant en 3 dimensions la pose de l'implant à partir d'images provenant de scanner ou d'IRM.

Les images 2D sont traitées au moyen d'un logiciel spécifique permettant de segmenter les zones osseuses d'intérêt pour la pause en ayant pour objectif de reconstruire une structure tridimensionnelle. Sur cette structure sont définis des repères permettant de déterminer un système de coordonnées spécifiques dans l'omoplate dans le cas du document précité.

Ce modèle tridimensionnel permet de simuler, planifier et mesurer précisément l'orientation de l'implant prothétique en fonction du système de coordonnées précitées.

L'opérateur planifie, non seulement, les tailles et positions de l'implant considéré mais compare également dans les 3 plans de l'espace la géométrie articulaire avec et sans prothèse en fonction des éléments choisis.

Autrement dit, le but recherché est de réaliser un gabarit de pose personnalisé au patient qui permet de restituer sur le patient la simulation faite à l'aide du logiciel de planification.

A partir de l'état de la technique défini par l'enseignement de ce document FR 2 967 047, le problème que se propose de résoudre l'invention est de réaliser un gabarit de pose sur mesure dans le cas d'un implant huméral.

L'invention est définie dans la revendication 1.

Il apparait que l'utilisation de la gouttière bicipitale s'avère particulièrement importante et intéressante pour le positionnement du gabarit en constituant pour l'opérateur une zone unique et facilement repérable.

A partir de cette conception de base, dans une forme de réalisation, l'embase d'appui et la partie constituant le moyen de préhension sont réalisées d'une manière monobloc.

Dans une autre forme de réalisation, l'une des pattes au moins est articulée.

La zone sécable est orientée angulairement par rapport à la patte coopérant avec la gouttière bicipitale et sensiblement perpendiculairement par rapport à la partie constituant le moyen de préhension.

Comme il ressort de l'enseignement de la demande de brevet précitée FR 2 967 047, le gabarit de pose selon l'invention résulte de la planification per opératoire 3D basée sur une reconstruction tridimensionnelle de l'humérus à partir de données tomodensitométriques et de repérages de points sur la tête épiphysaire de l'humérus, lesdits points étant projetés, d'une part, dans un plan dont la normale est l'axe diaphysaire et tangent à la calotte humérale et, d'autre part, dans un plan perpendiculaire au précédent et contenant certains points afin de calculer la rétroversion de l'humérus.

Avantageusement, les repérages des points sur l'humérus sont l'épicondyle médial, l'épicondyle latéral, le sommet de la tête humérale, le point bas de la tête humérale et le centre de la tête humérale.

Comme indiqué, l'invention trouve une application particulièrement avantageuse dans le cas d'une prothèse d'articulation d'épaule inversée.

L'invention est exposée ci-après plus en détails à l'aide des figures des dessins annexés dans lesquels :

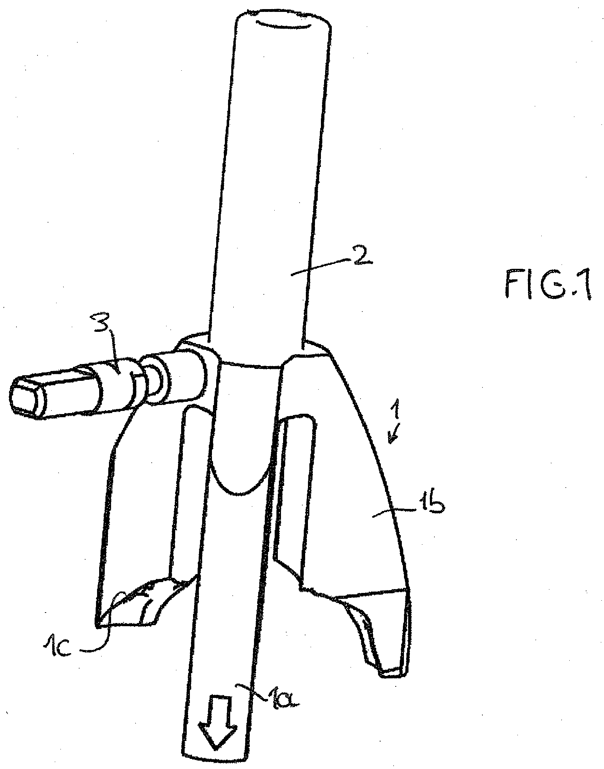

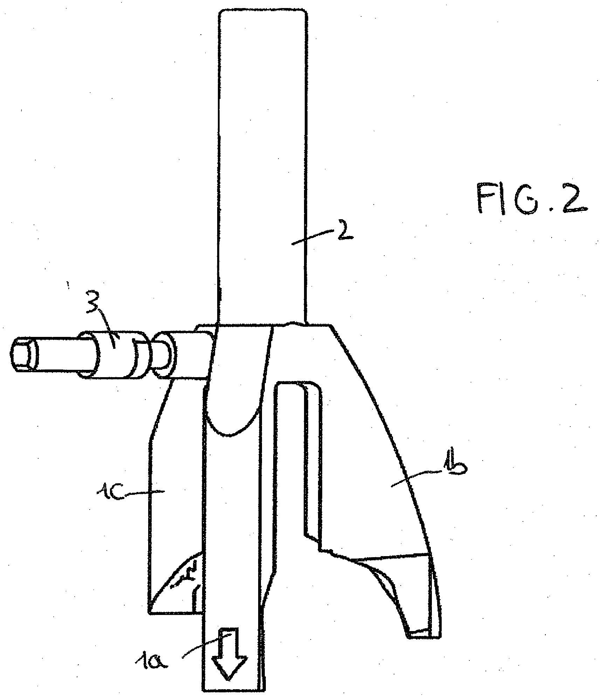

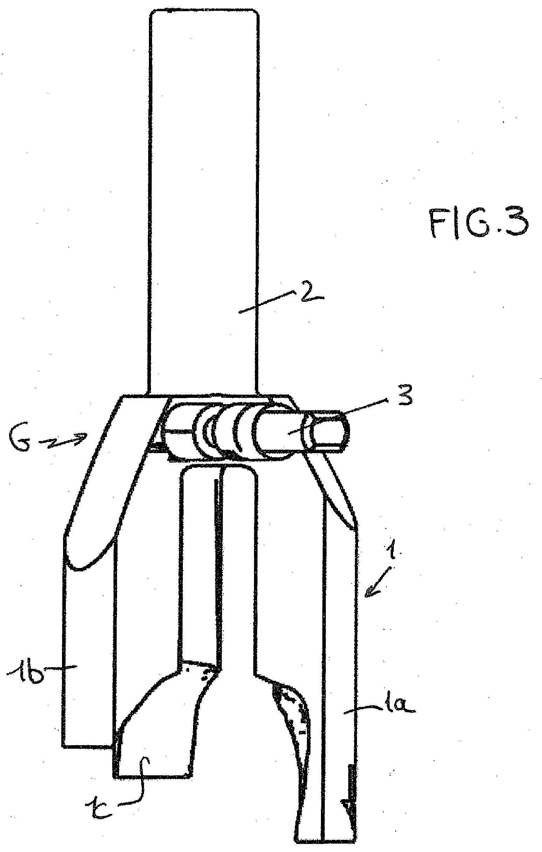

Les figures 1 à 3 sont des vues en perspective selon différentes orientations du gabarit de pose huméral,

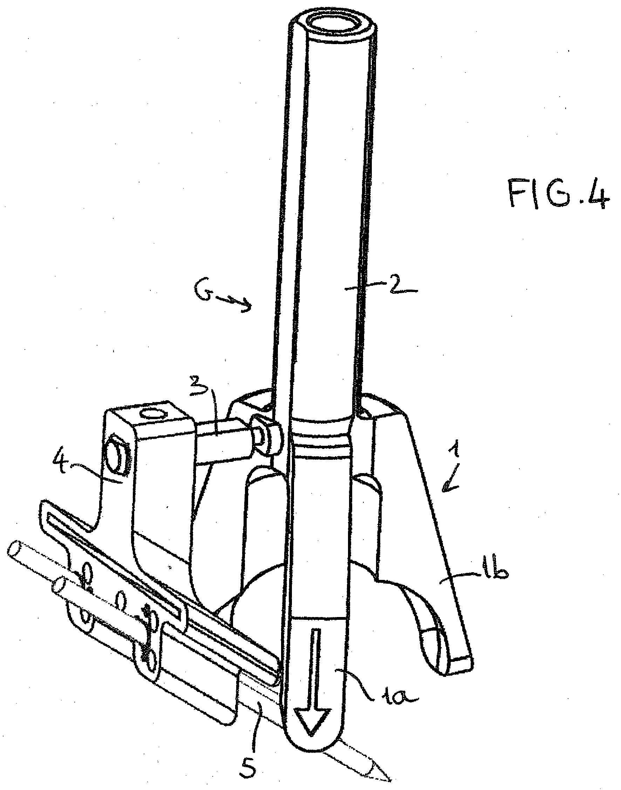

   - la figure 4 est une vue en perspective du gabarit de pose équipé d'un guide de coupe et de ses clous de fixation,

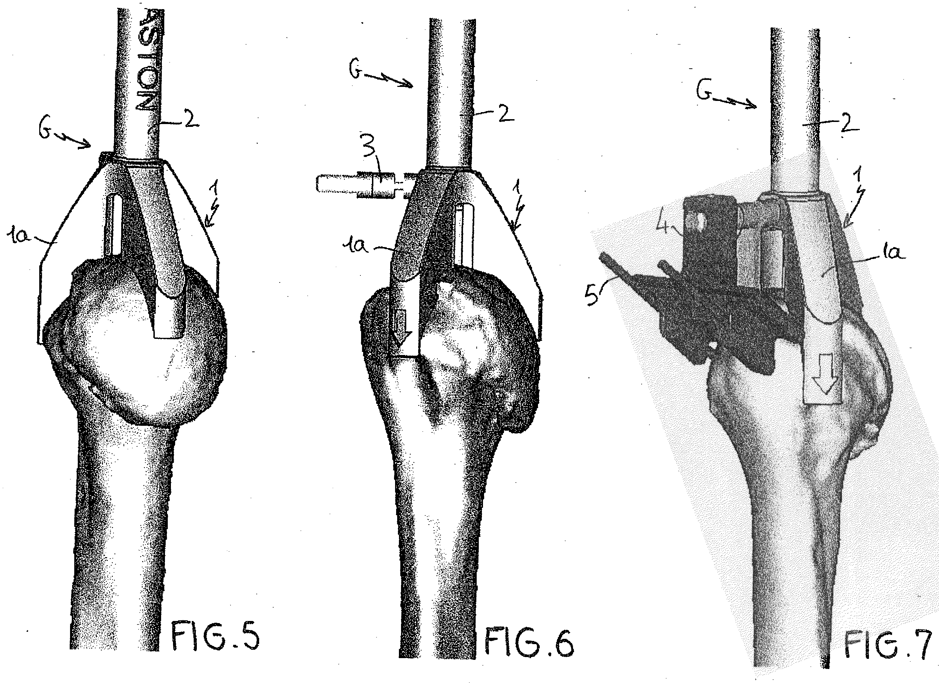

   - les figures 5 et 6 sont des vues en perspective montrant le positionnement du gabarit au niveau de la tête humérale,
   - la figure 7 est une vue en perspective correspondant à la figure 6 après mise en place du gabarit de coupe,
   - la figure 8 montre les points de repère sur l'humérus,
   - les figures 9, 10 et 11 montrent la définition des plans de l'humérus,
   - la figure 12 montre la rétroversion de l'humérus naturel,
   - la figure 13 montre la détermination du niveau de coupe humérale.

Comme indiqué, le gabarit de pose huméral sur mesure est basé sur une reconstruction tridimensionnelle des éléments osseux de l'articulation à partir de données tomodensitométriques, le repérage de points sur l'humérus servant de base au positionnement de l'élément prothétique considéré.

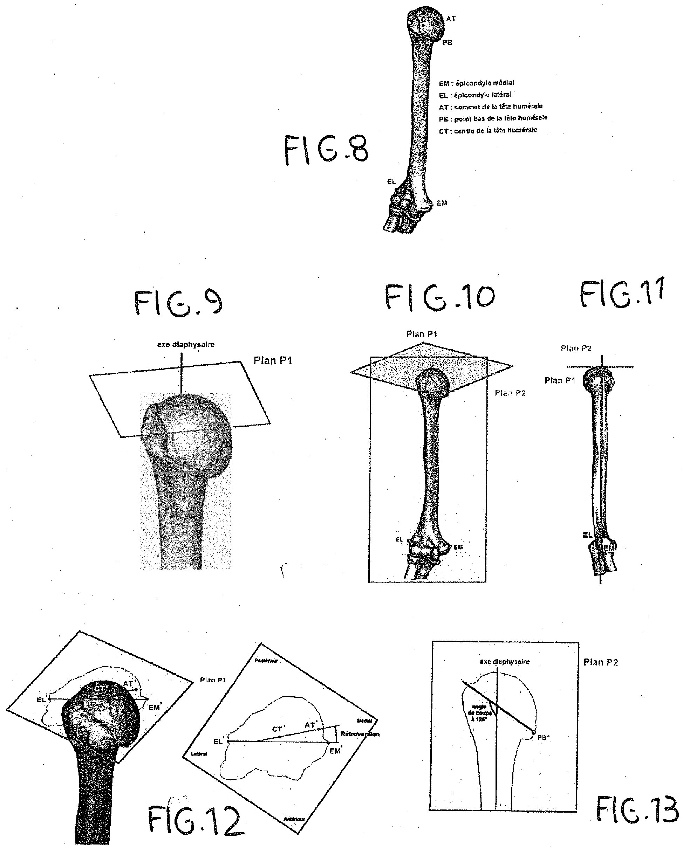

On renvoie à la figure 8 qui montre les points de repères sur l'humérus. (EM) correspond à l'épicondyle médial, (EL) à l'épicondyle latéral, (AT) au sommet de la tête humérale, (PB) au point bas de la tête humérale, (CT) au centre de la tête humérale.

Les figures 9, 10 et 11 montrent la définition des plans de l'humérus, à savoir, le plan (P1), dont la normale est l'axe diaphysaire et tangent à la calotte humérale et le plan (P2) perpendiculaire à (P1) et contenant (EL) et (EM).

La rétroversion de l'humérus naturel résulte de la projection des points EM, EL, CT, AT dans le plan P1 soit les points EM', EL', CT' AT' (figure 12).

La détermination du niveau de coupe humérale est définie par (TB") qui résulte de la projection du point (PB) dans le plan (P2).

Sur un écran de planification humérale, l'opérateur visualise une tige humérale en position dite « 0 » correspondant à une résection humérale de 125°, par exemple, passant par le point le plus bas de la calotte humérale et orientée sans rétroversion ni antéversion.

L'implant huméral est également, par défaut, en position neutre.

L'opérateur peut régler successivement la taille de la tige, la taille et la position de l'élément huméral ainsi que la rétroversion et la hauteur de la coupe humérale.

On procède ensuite à une approche mathématique de la position relative de l'ensemble osseux par rapport à l'ensemble prothétique planifié. A cet égard, cet écran donne un écart dans les 3 plans entre la position du centre articulaire planifié (centre de support de glène) et le centre articulaire en prothèse (centre de la tête humérale), l'écran donne également l'écart de latéralisation de l'humérus entre sa position initiale et sa position planifiée qui résulte d'une analyse réalisée avec un insert standard par défaut.

Il est possible pour l'opérateur de modifier cette latéralisation en faisant varier l'épaisseur de l'insert.

C'est à partir de cette planification qu'est réalisé le gabarit de pose huméral sur mesure qui permet de restituer en per-opératoire sur le patient la planification réalisée virtuellement avec le logiciel de planification.

Le gabarit de pose sur mesure désigné dans son ensemble par (G) selon l'invention, comprend une embase d'appui (1) coopérant avec la tête humérale selon une position unique et personnalisée.

Dans une forme de réalisation l'embase d'appui (1) forme un tripode et présente donc 3 pattes décalées angulairement (1a), (1b) et (1c), l'une des pattes (1a) coopérant avec la gouttière bicipitale de la tête humérale afin de constituer un repère unique pour l'opérateur.

Le gabarit (G) présente un moyen de préhension (2).

Dans une forme de réalisation, l'embase d'appui (1) et la partie (2) constituant le moyen de préhension, sont réalisées de manière monobloc, comme c'est le cas sur les figures des dessins annexés.

On n'exclut pas une autre forme de réalisation selon laquelle l'une des pattes au moins de l'embase d'appui (1) est articulée par rapport à la partie (2). La partie (2) se présente sous forme d'un manche qui peut présenter un alésage axial pour la mise en place d'une tige centromédullaire en cas de difficulté de mise en place du gabarit sur la calotte humérale.

Sensiblement au niveau du raccordement de l'embase d'appui (1) et du manche de préhension (2), le gabarit présente une partie sécable (3) apte à recevoir temporairement un bloc de coupe (4) pour permettre sa fixation à l'os huméral avant de permettre la réalisation de la résection humérale.

La zone sécable (3) est disposée entre la patte (1a) coopérant avec la gouttière bicipitale et l'une des autres pattes (1b) ou (1c).

Cette zone sécable (3) est orientée angulairement par rapport à la patte (1a) et sensiblement perpendiculaire à la partie constituant le moyen de préhension (2).

Après exposition de la tête de l'humérus, le gabarit de pose humérale selon l'invention est placé avant tout geste sur des ostéophytes en position unique grâce à la patte (1a) qui coopère avec la gouttière bicipitale.

Le bloc de coupe (4) est disposé sur la zone sécable (3) en butée sur le gabarit de sorte qu'il se trouve automatiquement positionné en hauteur et en orientation.

Le guide de coupe peut ensuite être fixé, par exemple, au moyen de clous (5) engagées dans la corticale du fémur. Après la fixation du bloc de coupe, il est possible de retirer le gabarit de pose en le cassant au niveau de la zone sécable (3), laquelle est ensuite enlevée du bloc de coupe.

Dans la forme de réalisation illustrée, la zone sécable (3) est constituée par une portée circulaire sur laquelle peut être librement engagée une partie du guide de coupe.

La zone sécable en tant que telle résulte d'un amincissement de la section de la portée circulaire au niveau de son raccordement avec le guide de pose.

Après avoir retiré le gabarit et en laissant en place le guide de coupe comme indiqué, il est possible de procéder à la résection humérale dans la position planifiée en per opératoire au moyen du logiciel de planification et restitué en per opératoire par le gabarit sur mesure (G).

De manière avantageuse le gabarit de pose huméral selon l'invention remplace la tige centro-médullaire de positionnement du guide de coupe selon l'état antérieur de la technique.

Dans une forme de réalisation en variante, le gabarit et le bloc de coupe peuvent être monobloc pour constituer un ensemble jetable à usage unique.

Les avantages ressortent bien de la description.

## Revendications

1. Gabarit de pose d'un implant d'articulation d'une prothèse d'épaule sur une tête humérale, ledit gabarit est réalisé sur la base d'une reconstruction tridimensionnelle des éléments osseux de l'articulation à partir de données tomodensitométriques et de repérages de points sur l'humérus servant de base au positionnement de l'élément d'articulation considéré, le gabarit présentant :
- une partie (2) constituant un moyen de préhension, et
- une zone sécable (3) apte à recevoir temporairement un bloc de coupe (4) pour permettre sa fixation à l'os huméral avant de permettre la réalisation de la résection humérale, ***caractérisé* en ce que** le gabarit présente :
- une embase d'appui (1) formant un tripode avec trois pattes décalées angulairement (1a), (1b), (1c), dont l'une (3a) coopère avec le sillon interbuberculaire ou gouttière bicipitale de la tête humérale pour constituer un repère unique ladite zone sécable (3) recevant le bloc de coupe (4) étant disposée entre la patte (1a) coopérant avec la gouttière bicipitale et l'une des deux autres pattes (1b) ou (1c).

2. Gabarit de pose selon la revendication 1, ***caractérisé* en ce que** l'embase d'appui (1) et la partie constituant le moyen de préhension (2) sont réalisées d'une manière monobloc.

3. Gabarit de pose selon la revendication 1, ***caractérisé* en ce que** l'une des pattes au moins est articulée.

4. Gabarit de pose selon la revendication 1, ***caractérisé* en ce que** la zone sécable (3) est orientée angulairement par rapport à la patte (1a) coopérant avec la gouttière bicipitale et sensiblement perpendiculairement par rapport à la partie constituant le moyen de préhension.

## Patentansprüche

1. Eine Schablone zum Einsatz eines Gelenkimplantates einer Schulterprothese auf einen Humeruskopf, wobei die besagte Schablone auf der Grundlage einer dreidimensionalen Rekonstruktion der Knochenelemente des Gelenks ab den computer-tomographischen Daten und den Referenzpunkten auf dem Humerus, die als Basis zur Positionierung des betrachteten Gelenkelements gedient haben, und wobei die Schablone folgendes umfasst
- Einen Teil (2), der ein Greifmittel bildet, und
- Einen abtrennbaren Abschnitt (3), der geeignet ist, temporär einen Schneidführungsblock (4) aufzunehmen, um seine Befestigung am Humerusknochen zu ermöglichen, bevor die Humeral-Resektion ermöglicht wird, **dadurch gekennzeichnet, dass** die Schablone folgendes aufweist:
- einen Stützansatz (1), der einen Tripod mit drei winkel-versetzten Füßen (1a), (1b), (1c), von denen eine (3a) mit der Zwischenhöckerrinne des Humeruskopfes kooperiert, um einen einzigen Referenzpunkt zu bilden, wobei besagter abtrennbarer, und den Schneidführungsblock (4) aufnehmender Abschnitt (3) zwischen dem mit der Zwischenhöckerrinne des Humeruskopfes kooperierenden Fuß (1a) und einem der beiden anderen Füße (1b) oder (1c) angeordnet ist.

2. Schablone gemäß Anspruch 1, **dadurch gekennzeichnet, dass** der Stützansatz (1) und der Teil, der das Greifmittel (2) bildet, auf Art eines Monoblocks angefertigt sind.

3. Schablone gemäß Anspruch 1, **dadurch gekennzeichnet, dass** mindestens einer der Füße angelenkt ist.

4. Schablone gemäß Anspruch 1, **dadurch gekennzeichnet, dass** der abtrennbare Abschnitt (3) in Bezug auf den der Zwischenhöckerrinne des Humeruskopfes kooperierenden Fuß (1a) abgewinkelt und spürbar senkrecht in Bezug auf den Teil, der das Greifmittel bildet, orientiert ist.

## Claims

1. A installation template for a joint implant of a shoulder prosthesis on a humeral head, said template is made on the basis of a three-dimensional reconstruction of the osseous elements of the jointe from tomodensitometric data and markings of points on the humerus useful for the positioning of the relevant joint element, the template comprising:
- a part (2) forming gripping means, and
- a breakable area (3) capable of temporarily receiving a cutting block (4) for its fixing to the humeral bone before allowing the implementation of the humeral resection, **characterized in that** the template comprises:
- a support base (1) forming a tripod with three angularly shifted legs (1a), (1b), (1c), one of whom (3a) cooperating with the interbuberculaire groove or bicipital groove of the humeral head to constitute a single mark, said breakable area (3) receiving the cutting block (4) while being arranged between the leg (1a) cooperating with the bicipital groove and one of the two others legs (1a) or (lb).

2. The installation template according to claim 1, **characterized in that** the supporting base (1) and the part forming the gripping means (2) are made of a single piece.

3. The installation template according to claim 1, **characterized in that** at least one of the legs is hinged.

4. The installation template according to claim 1, **characterized in that** the breakable area (3) is angularly oriented with respect to the leg (1a) cooperating with the bicipital groove, and substantially perpendicular with respect to the part forming the gripping means.
